# EUROPEAN PATENT SPECIFICATION

(11) **EP 0 479 301 B1**
(45) Date of publication and mention of the grant of the patent: **31.07.1996**
(21) Application number: 91116890.4
(22) Date of filing: 03.10.1991
(51) Int. Cl.: C12N 15/80, C12N 1/14

(54) **A host vector system**
Wirt-Vektor-System
Système hôte-vecteur

(30) Priority: 04.10.1990 JP 267357/90; 04.03.1991 JP 62693/91
(43) Date of publication of application: 08.04.1992
(73) Proprietor: Nihon Shokuhin Kako Co., Ltd., Chiyoda-ku Tokyo (JP)
(72) Inventor: Takagi, Masamichi, Fuchu-shi, Tokyo (JP); Horiuchi, Hiroyuki, Tokyo (JP); Yanai, Koji, Urawa-shi, Saitama-ken (JP); Sakaguchi, Kenji, Tokyo (JP)
(74) Representative: Klunker . Schmitt-Nilson . Hirsch

(56) References cited:
- WO-A-86/03774
- WO-A-90/00192
- WO-A-91/00920
- CURRENT GENETICS vol. 19, no. 3, 1991, BERLIN, G pages 221 - 226; K. YANAI ET AL.: 'Transformation of Rhizopus niveus using a bacterial blasticidin S resistance gene as a dominant selectable marker'
- AGRICULTURAL AND BIOLOGICAL CHEMISTRY. vol. 54, no. 10, October 1990, TOKYO JP pages 2689 - 2696; K. YANAI ET AL.: 'Preparation of protoplasts of rhizopus niveus and their transformation with plasmid dna'
- WORLD PATENTS INDEX LATEST Week 9014, Derwent Publications Ltd., London, GB; AN 90-103113
- WORLD PATENTS INDEX LATEST Week 8902, Derwent Publications Ltd., London, GB; AN 89-011781
- MGG (MOLECULAR & GENERAL GENETICS) vol. 212, no. 1, 1988, BERLIN, G pages 120 - 123; T. SUAREZ ET AL.: 'Transformation of Phycomyces with a bacterial gene for kanamycin resistance'
- BIOTECHNOLOGY ABSTRACTS, Derwent Publications London, GB, Abstract no 91-00674 & JP-A-2231080 (YAKULT-HONSHA) 13 September, 1990

## Description

### BACKGROUND OF THE INVENTION

### Field of the invention

The present invention relates to a novel host vector system in which hosts are Rhizopus and vectors are derived from Mucor circinelloides.

### Description of the related art

A method for preparing useful proteins such as interferon, interleukin and others by genetically engineered bacteria has been established. However, problems are now occurring in the method using bacteria as hosts. For example, the extracellular secretion of the proteins is not so efficient in many bacteria which is sometimes very necessary for the production of large amount of target proteins. Moreover, the intracellularly produced interferon or interleukin prepared by E. coli has the wrong secondary structure which is different from that of the natural protein. As a result, the artificial protein is recognized in a human body as an antigen. Thus denaturation and renaturation procedures of the artificial protein are required prior to the pharmaceutical use of the protein.

Another important advantage of fungal genetic engineering system is its ability to produce glycosylated protein which is impossible with bacterial host vector systems. Many human and animal hormones and other physiologically important proteins are attached with oligosaccharide chains. These glycomoieties are sometimes indispensable for the physiological functions of the human hormones as in the case of erythropoietin, and in many cases increase the stability of target proteins against the attack of proteinases in the culture medium, in the injected body fluids, ect.. Each fungi and yeasts produce each different structures of glycomoieties, which gives different recognition signals to the human immunological systems and others. Therefore, the construction of various fungal genetic systems are important. The host vector systems using animal and human cells as hosts produce similar or correctly glycosylated proteins, however, their production ability of the target proteins are much lower than the fungal system.

Phycomycetes such as Rhizopus or Mucor are used frequently in the fermentation technology. They are classified into Phycomycetes. Rhizopus has the extensive ability to secrete enzymes and proteins extracellularly. For instance, the amount of glucoamylase secreted by Rhizopus niveus into the liquid culture medium is about 20 g/liter and that secreted with solid culture procedure is about 30 g/kg. Further, not only the primary structure but the secondary structure of the resulting protein is the same as that of a natural protein due to the extracellular secretion system through membrane. Therefore, it is expected that transformed molds will be used for the preparation of various kinds of commercially important enzymes and proteins. However, only a few transformation techniques are established for fungi and any transformation technique used for Rhizopus has not been known yet.

WO-A-86/03774 discloses a method of transforming a fungus of the class Zygomycetes, in particular a fungus of the genus Mucor, with a recombinant expression vector which is replicated in the fungal species. For the transformation vectors derived from the same genus as the host fungus are used. The transformed fungi may allow production of gene products like hormones, nucleic acids or enzymes.

JP-A-2053480 discloses a method of transforming a fungus of the genus Rhizopus, e.g. Rhizopus niveus. The respective protoplast cells are treated with polyethyleneglycol in the presence of objective protein coding DNA fragments or plasmids containing the DNA, in order to obtain fused cells containing the desired DNA.

T. Suarez et al, 1988, Mol. Gen. Genet., Vol. 212, pages 120-123, relates to the transformation of Phycomyces protoplasts for kanamycin resistance. Transformants could be obtained with a plasmid having a Phycomyces insert.

Under the circumstances, the present inventors successfully provided a transformation technique used for Rhizopus (see Japanese Patent Disclosure (KOKAI) (JP-A-) 2-53480).

However, a vector suitable for producing an enzyme by using Rhizopus as a host has not been known.

An object of the present invention is to provide a host vector system suitable for the production of proteins and enzymes using Rhizopus as a host which causes the enhanced ability to produce proteins and enzymes extracellularly and in the cell because of their large cell mass production.

### SUMMARY OF THE INVENTION

The present invention relates to a host vector system comprising Rhizopus species and a plasmid derived from Mucor circinelloides wherein said plasmid contains an origin of replication from pLeu 4 and is capable of transforming said Rhizopus species in order to produce a desired target protein.

### BRIEF DESCRIPTION OF THE DRAWINGS

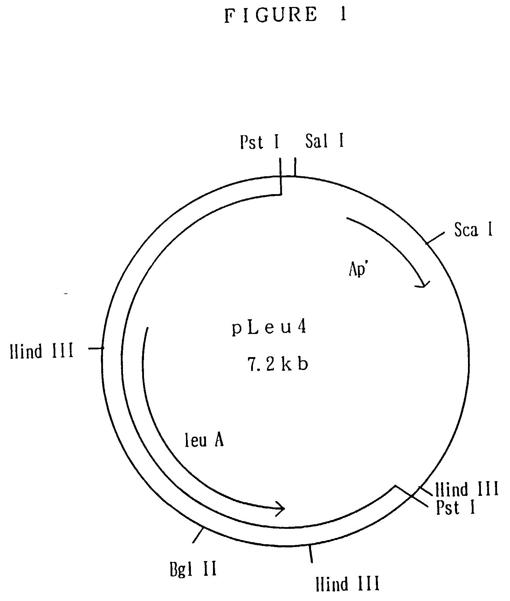

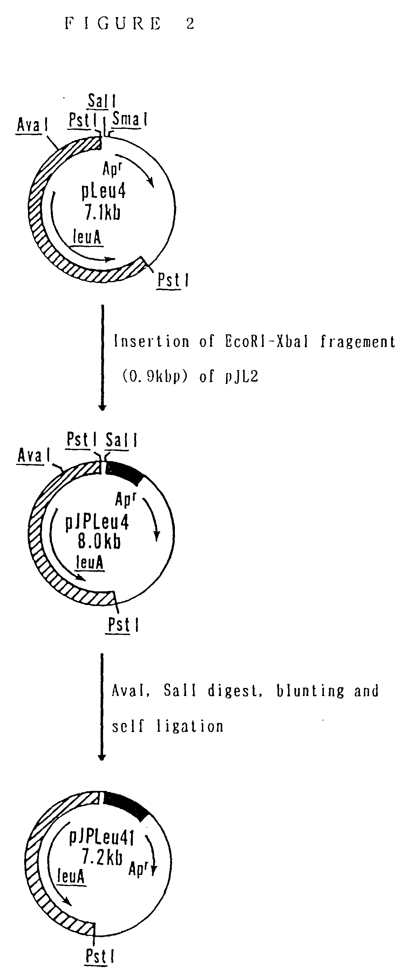

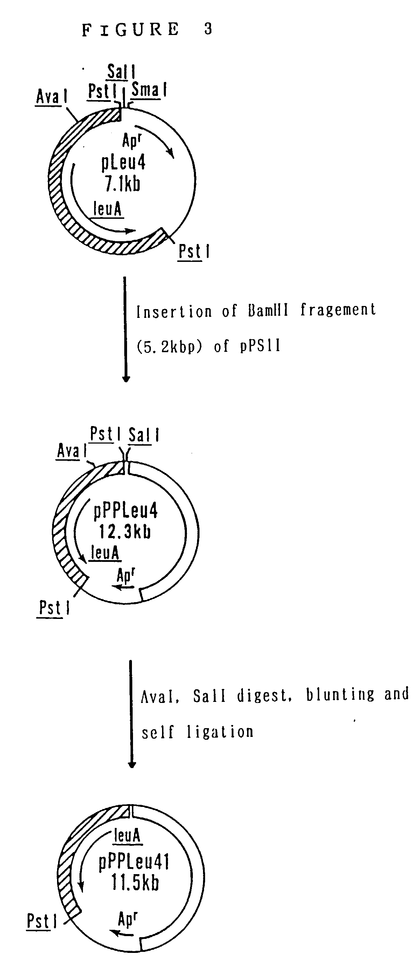

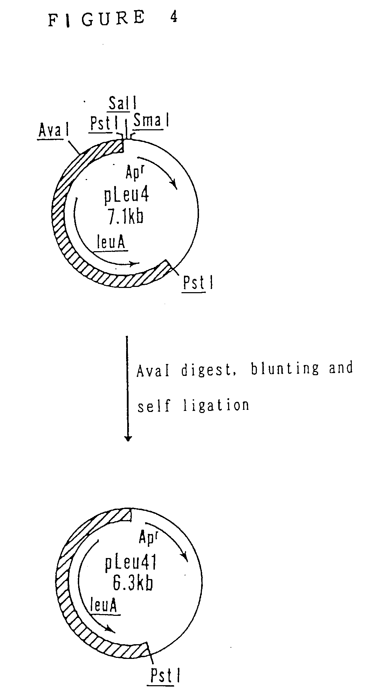

Figure 1 illustrates a restriction map of plasmid pLeu4. Figures 2 and 3 illustrate a preparation method of plasmids pJPLeu4 and pJPLeu41. Figure 4 illustrates a preparation method of plasmid pLeu41.

### DETAILED DESCRIPTION OF THE INVENTION

Filamentous fungi Rhizopus niveus (IF04810) is used as a host of the host vector system of the invention. Further, Rhizopus species can be selected from the group consisting of Rhizopus delemar such as ATCC 9374 strain, Rhizopus javanicus such as ATCC 22581 and 22580 strains, Rhizopus oligosporus such as ATCC 22959 strain, Rhizopus nigricans such as ATCC 24862 strain and Rhizopus oryzae such as JCM 5557=IAM 6015 and JCM 5558=IAM 6019 strains.

A plasmid derived from Mucor circinelloides is a vector of the system of the invention. The use of the plasmid derived from Mucor circinelloides as the vector enables to transform the host, Rhizopus niveus.

Examples of the plasmid derived from Mucor circinelloides include plasmids pLeu4, pPS11, pMA67, pJL1, pJL2, pMCL1302, pMCL002, pJPLeu4, pPPLeu4, pLeu41, pJPLeu41 and pPPLeu41 (cp. figures 2-4).

The above-mentioned plasmids are obtainable according to the following methods.

Plasmid pLeu4 is obtainable in accordance with the method reported in the article by Roncero, M.I.G. et al, Gene, 84, 335-343 (1989). The disclosure of which is hereby incorporated by reference.

Plasmid pPS11 is obtainable from Phycomyces blakesleeanus (NRRL1555 strain) according to the method reported in the article by T. Suarez et al, Mol. Gen. Genet. 212, 120-123, 1988. The disclosure of which is hereby incorporated by reference.

Plasmid pMA67 is obtainable from plasmid pMCL1302 according to the method reported in the article by L. Dickinson et al, Carsberg Research Communications, 52, 243-252, 1987. The disclosure of which is hereby incorporated by reference.

Plasmids pJL1 and pJL2 are obtainable from Phycomyces blakesleeanus (A459 strain) according to the method reported in the article by J.L. Revuetta et al, Proc. Nat. Acad. Sci U.S.A., 83, 7344-7347, 1986. The disclosure of which is hereby incorporated by reference.

Plasmids pMCL1302 and pMCL002 are obtainalbe from Mucor circinelloides f. lusitanicus (CBS227.49 strain) which is the same as Mucor racemosus (ATCC 12166 strain) according to the method reported in the article by R. van Heeswijck, Carsberg Research Communications, 51, 433-443, 1986. The disclosure of which is hereby incorporated by reference.

Plasmid pJPLeu4 is obtainable by insertion of an EcoRI-XbaI fragment (0.9 kbp) of plasmid pJL2 into a SamI site of pLeu4. pJPLeu41 is obtainable by digestion of plasmid pJPLeu4 with restriction enzymes AvaI and SalI, and self ligation of the fragment of 7.2 kbp obtained by the digestion.

Plasmid pPPLeu4 is obtainable by insertion of a BamHI fragment (5.2 kbp) obtained from plasmid pPSll into a SamI site of pLeu4. Plasmid pPPLeu41 is obtainable by digestion of plasmid pPPLeu4 with restriction enzymes AvaI and SalI, and self ligation of the fragment of 11.5 kbp obtained by the digestion.

Plasmid pLeu41 is obtainable by digestion of plasmid pLeu4 with restriction enzymes AvaI and SalI, and self ligation of the fragment of 6.3 kbp obtained by the digestion.

The above mentioned vector(s) is inserted into a chromosome(s) of a host(s) Rhizopus niveus, or contained in a cytoplasm(s) of Rhizopus niveus. Further it is possible that the vector(s) is inserted into a chromosome(s) of Rhizopus niveus and contained in a cytoplasm(s) of Rhizopus niveus simultaneously. When the vector(s) is inserted into the chromosome(s) of Rhizopus niveus, the number of the vector(s) inserted into a chromosome is one or more. When the vector(s) is contained in the cytoplasm(s) of Rhizopus niveus, the number of the vector(s) contained in a cytoplasm of is also one or more.

The host vector system of the present invention can be prepared based on the method mentioned in Japanese Patent Disclosure (KOKAI) (JP-A-) 2-53480. The disclosure of which is hereby incorporated by reference.

Spores of Rhizopus niveus are cultured to obtain germination tubes. The germination tubes, spores or mycelia are treated with Novozym 234, chitinase and chitosanase to obtain protoplast cells. The cells are treated with polyethylene glycol in the presence of plasmids of Mucor circinelloides to obtain fused cells containing the plasmids of Mucor circinelloides. The host vector system of the present invention is obtained by subjecting the fused cells to renaturation.

The plasmids of Mucor circinelloides are cyclic and can be used as they are. Alternatively, the plasmids are in a linear DNA(s) form which are obtained by cutting the plasmid with a suitable restriction enzyme(s). There is a tendency that the rate of transformation is high when the cyclic plasmids are used and that the insertion rate of the plasmid(s) into a chromosome is high when the linear one is used.

### Examples

The present invention is illustrated more in detail in reference to the following examples.

### Example 1

### Preparation of auxotrophic mutant

1 ml of a suspension solution containing 10⁶ - 10⁷ pieces/ml of spores of Rhizopus niveus was irradiated with UV ray (0.0036 J/cm² ) and the viability was measured. The solution was applied on a plate complete agar medium. After culturing at 37°C for about 20 days, spores were collected and washed with a saline. Then the resulting spores were subjected to stationary culture in a liquid minimal medium at 30°C and filtered with a G3 glass filter. These procedures were repeated until the spores no longer grew.

Thus obtained spores were suspended in a saline, and the suspension was applied on a plate acid complete agar medium (2.4 % potato dextrose (availabe from Difco / USA), 0.1 % HCl, 1.5 % agar) and cultured at 30 °C for 2 - 3 days. After culturing, the spores on the medium were replicated to a minimal agar medium (2 % glucose, 0.2 % asparagine, 0.05 % KH₂PO₄, 0.025 % MgSO₄ · 7H₂O, 1.5 % purified agar) containing various amino acids and nucleic acids.

A leucine (leu) auxotrophic mutant was selected from the replicated spores by a general manner to obtain R. niveus M 37 strain.

### Transformation of Rhizopus niveus

2 - 5 x 10⁷ pieces of the resulting R. niveus M 37 strain, a leu auxotrophic mutant, were suspended in water and filtered with a G1 glass filter. The resultant filtrate was filtered with a G3 glass filter. Thus obtained filtrate was centrifuged at 2500 rpm for 7 minutes. The precipitate was suspended in 5 - 8 ml of a solution containing 1 % glucose, 20 % yeast extract, 2 % polypeptone and 0.01 M proline and subjected to a shaking culture with a reciprocal shaker at 300 rpm at 30 °C for 4.5 to 5 hours. After the formation of germination tubes was confirmed by the observation with a microscope, the suspension was filtered with a G1 glass filter and the filtrate was centrifuged at 3000 rpm for 3 minutes. The precipitate was suspended in 5 ml of buffer solution (A) (13.2 mM citric acid, 33 mM Na₂HPO₄, 0.3 M mannitol) and centrifuged at 3000 rpm for 3 minutes. The precipitate was suspended in buffer solution (A) again and centrifuged at 3000 rpm for 3 minutes.

The resulting precipitate was suspended in 2 ml of buffer solution (A). 35 mg of Novozym 234 (available from Novo corporation in Denmark), 14 mg of chitinase ABC (available from Advanced Biofactures/ USA ) and 10 units of chitosanase (available from Wako in Japan) were suspended in 5 ml of buffer solution (A). The suspension was added to the precipitate suspension and subjected to a shaking culture by the use of a reciprocal shaker (at 60 rpm) at 30 °C for 1.5 to 2 hours. After the formation of protoplast cells was confirmed by observation with a microscope, the suspension was filtered with a G2 glass filter. The filtrate was centrifuged at 450 rpm for 4 minutes. The precipitate was suspended in 5 ml of buffer solution (B) (10 mM MOPS (pH 6.3), 50 mM CaCl₂, 0.3 M mannitol) and centrifuged at 450 rpm for 4 minutes. These suspension and centrifugation procedures were repeated twice The precipitate was suspended gradually in 200 µl of buffer solution (B) and mixed with 10 µl of a plasmid pLeu4 (Roncero, M.I.G. et al, Gene, 84, 335-343(1989); plasmid pLeu4 has a leuA gene of Mucor circinelloides and an ARS element working in Mucor circinelloides) suspension prepared by suspending about 10 to 20µ g of pLeu4 in 10µ l of a solution containig 10 mM MOPS (pH 6.3), 50 mM CaCl₂ and 1 mg/20µ l of heparin. The mixture was allowed to stand for 5 minutes in an ice bath and 10 µ l of buffer solution (C) (10 mM MOPS (pH 6.3), 50 mM CaCl₂, 40 % PEG 4000 solution) was added. The mixture was allowed to stand for 25 minutes in an ice bath and 1.25 ml of buffer solution (C) was further added. Then the mixture was allowed to stand for 25 minutes at room temperature. The mixture was mixed with 10 ml of buffer solution (B) and centrifuged at 600 rpm for 3 minutes.

The precipitate was suspended in 1 ml of a solution containing 1 % glucose, 2 % yeast extract, 2 % polypeptone and 0.3 M mannitol. The suspension was transferred to an Eppendrof tube and subjected to the stationary culture at 30°C for 30 minutes. Then the suspension was centrifuged at 1000 rpm for 1 minute. The precipitate was suspended in 1 ml of 0.4 M mannitol solution and centrifuged at 1000 rpm for 1 minute. These suspension and centrifugation procedures were repeated twice. The precipitate was suspended in 100 µ l of 0.4 M mannitol solution and mixed with 5 ml of medium (D) (SIV minimal medium, 0.35 M mannitol and 0.2 % H₂SO₄ were dissolved at 48°C ) containing 1 % purified agar. Then the mixture was placed on a plate medium (D) containig 1.5 % purified agar to form a layer and cultured at 30 °C for 2 to 3 days. Each colony was replicated to SIV minimal medium and stored. That is, plasmid pLeu4 was complementary to R. niveus M 37 strain being leu auxotroph and it was clarified that plasmid pLeu4 was functionable in R. niveus M 37 strain as a vector.

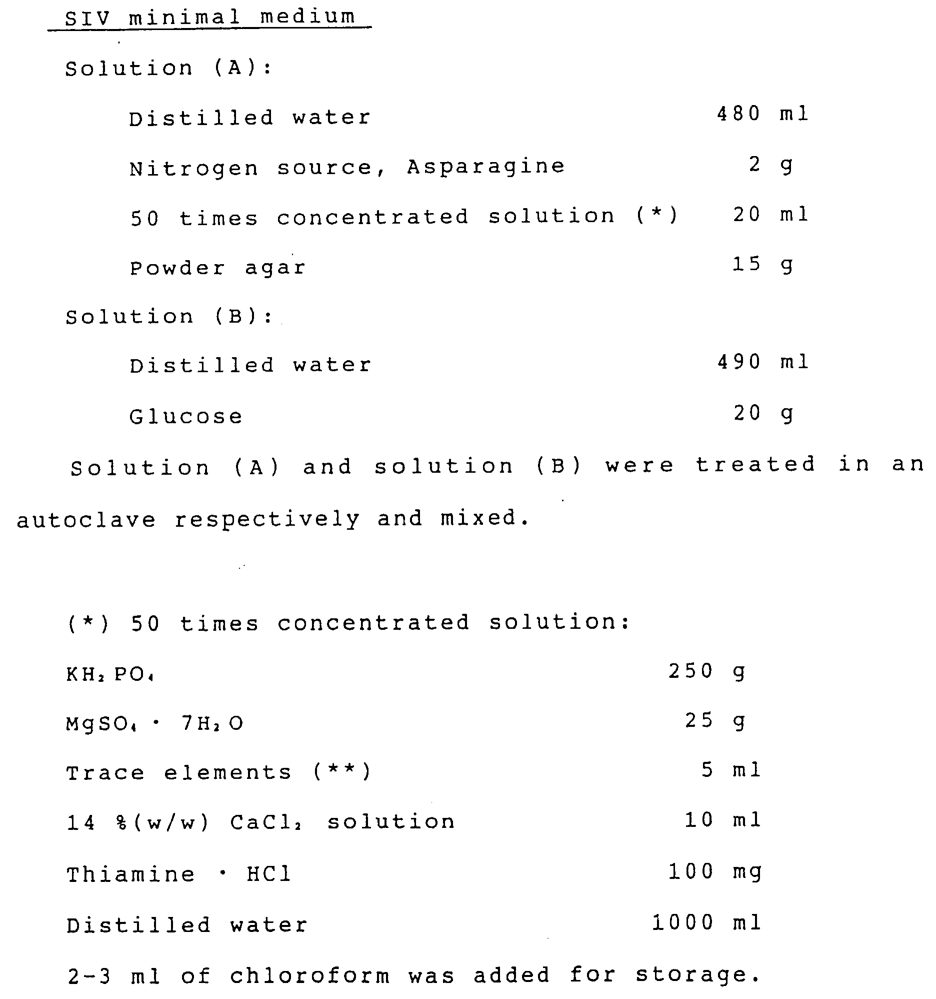

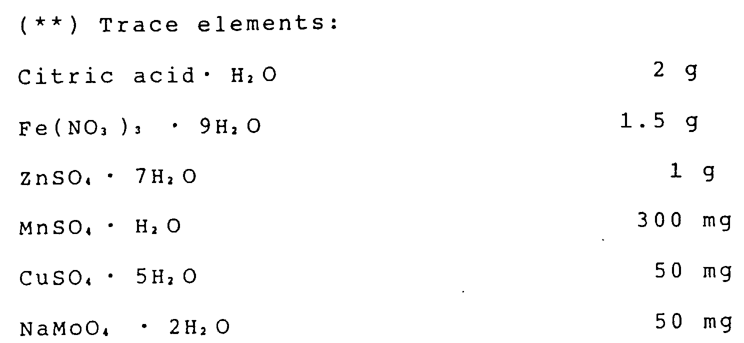

All DNAs were extracted from the obtained transformants and subjected to an analysis by southern blot technique. As a result, plasmid pLeu4 was inserted into a chromosome of Rhizopus niveus M 37 strain in the form of tandem or contained in a cytoplasm of Rhizopus niveus M 37 strain.

E. coli was transformed by using all DNAs extracted from the obtained transformants. Then the plasmid pLeu4 was recovered in its complete form from the transformed E. coli cells. This means that plasmid pLeu4 contained the ARS activity and was replicable in Rhizopus niveus M 37 strain.

### Example 2

With the same procedures described in Example 1, the linear DNA fragments obtained by cutting the cyclic plasmid pLeu4 with each one of 4 kinds of restriction enzymes (Pst I, Bgl II, Sac I and Sca I) were transformed into Rhizopus niveus. The cyclic plasmid pLeu4 was the same as that used in Example 1 and of which restriction map is shown in Figure 1 . As a result, leu auxotrophy was compensated by use of every fragment. All DNAs of the obtained transformants were subjected to an analysis by southern blot technique. As a result, ratio of plasmids inserted into chromosomes was higher than that of the transformation using cyclic plasmids.

### Example 3

Rhizopus niveus was plated on a PD medium or a minimal medium and cultured at 30 °C for about 10 days to obtain spores. Conidiophores were collected from each mycelium and suspended in 30 ml of sterilized water. Then the water was stirred strongly to release spores included in the conidiophores. The spores were collected by a G3 glass filter from the resulting suspension. The spore suspension was centrifuged at 3000 rpm at room temperature for 10 minutes to collect spores. The spores were washed with a saline and suspended (2-5x10⁷ pieces/ml) in a YPG liquid medium (1 % glucose, 2 % polypeptone, 2 % yeast extract). The spore suspension was poured into a sterilized test tube with a cotton plug and subjected to a shaking culture at 30 °C for about 5 hours (a reciprocal shaker at 300 rpm) until germination tubes were formed. Then spores excessively germinated were removed from spores immediately after germination. The filtrate was centrifuged and the precipitate was washed with 0.3 x McIlvaine buffer solution (13.3 mM citric acid, 33 mM Na₂HPO₄, pH 5.6) twice to substitute the buffer solution.

The germinated spores were suspended in 7 ml of the above mentioned buffer solution containing 5 mg/ml of Novozym 234 (available from Novo corporation in Denmark), 2 mg/ml of chitinase ABC (available from Advanced Biofactures/ USA ) and 1.52 mg/ml of chitosanase (available from Wako in Japan). The resulting suspension was added to a plastic tube and subjected to a shaking culture at 40 rpm at 30 °C for 1.5 to 2 hours. The residue of protoplasted cells was removed and the filtrate was subjected to centrifugation by a swing rotor (70 x g) for 4 minutes to collect protoplast cells. The collected cells were washed with buffer solution (A) twice and suspended in 400µ l of buffer solution (A) (10 mM MOPS (pH 6.3), 50 mM CaCl₂, 0.3 M mannitol). 100 µ l of the resulting solution was mixed with a plasmid DNA solution which was prepared in advance by dissolving plasmid pLeu4 in 10 µ l of buffer solution (C) (buffer solution (A) added with heparine in the concentration of 50mg/ml) and being allowed to stand in an ice bath for 20 minutes or more. The mixture was allowed to stand in an ice bath for 5 minutes, and 10 µ l of 40 % PEG solution (10 mM MOPS (pH 6.3), 50 mM CaCl₂, 40 % PEG 4000 solution) was added. The mixture was mixed slowly and allowed to stand for 25 minutes in an ice bath.

1.25 ml of the 40 % PEG solution was further added. Then the mixture was allowed to stand for 25 minutes at room temperature. The mixture was mixed with 10 ml of buffer solution (A) and was subjected to centrifugation by a swing rotor (50 x g) for 4 minutes to collect cells. 1 ml of a YPG medium containing 0.3 M mannitol was added to the collected protoplast cells and allowed to stand at 30 °C for 30 minutes. Then the protoplast cells were washed with 0.4 M mannitol solution twice and plated on a minimal medium. The number of generated colonies was counted and the colony number per 1 µ g g of DNA used was calculated. The results were listed in Tables 1 and 2.

**Table 1**

| Plasmid | transformation frequency (cfu/ µ g) | | |
|---|---|---|---|
| | ex 1 | ex 2 | ex 3 |
| pLeu4 | 0.56 (1) | 2.1 (1) | 0.2 (1) |
| pJLeu4 | 1.52 (2.71) | 6.85 (3.26) | 0.6 (3) |
| pPPLeu4 | 10.4 (1.85) | 7.7 (3.66) | 1.3 (6.5) |

In ex 1, ex 2 and ex 3, 25, 20 and 10µ g of plasmid DNA was used respectively.

**Table 2**

| Plasmid | transformation frequency (cfu/ µ g) | | |
|---|---|---|---|
| | ex 1 | ex 2 | ex 3 |
| pLeu4 | 2.2 (1) | 1.5 (1) | 3.6 (1) |
| pLeu41 | 3.95 (1.79) | 2.0 (1.33) | 4.8 (1.33) |
| pJPLeu41 | 8.55 (3.88) | 4.6 (3.07) | 7.1 (1.97) |
| pPPLeu41 | 6.7 (3.04) | 3.1 (2.07) | N.T. |
| In ex 1, ex 2 and ex 3, 20, 10 and 20 µg of plasmid DNA was respectively used. | | | |

| | | | |
|---|---|---|---|
| N.T.: not tested | | | |

Plasmid DNA used in the above experiments was prepared in accordance with the flow charts shown in Figures 2 to 4 as explained below.

pJPLeu4: An EcoRI-XbaI fragment (0.9 kbp) was isolated from plasmid pJL2 and the termini of the fragment was made smooth with T4 polymerase. The fragment was ligated by using T4 DNA ligase to plasmid pLeu4 which was previously digested with Sam I and dephosphorylated to obtain plasmid pJPLeu4.

pJPLeu41: Plasmid pJPLeu41 was obtained by digesting plasmid pJPLeu4 with Ava I and Sal I, blunting the termini of the resulting 7.2 kbp fragment with T4 polymerase and self ligating the fragment.

pPPLeu4: BamHI fragment (5.2 kbp) was isolated from plasmid pPS11 and the termini of the resulting 7.2 kbp fragment was made smooth with T4 polymerase. The fragment was ligated by using T4 DNA ligase to plasmid pLeu4 which was previously digested with Sam I and dephosphorylated to obtain plasmid pJPLeu4.

pPPLeu41: Plasmid pPPLeu41 was obtained by digesting pPPLeu4 with AvaI and SalI, blunting the termini of the resulting 11.5 kbp fragment with T4 polymerase and self ligating the fragment.

pLeu41: Plasmid pLeu41 was obtained by digesting pLeu4 with AvaI and SalI, blunting the termini of the resulting 6.3 kbp fragment with T4 polymerase and self ligating the fragment.

## Claims

1. A host vector system for the production of a target protein in Rhizopus, said system comprising a Rhizopus species and at least one plasmid derived from Mucor circinelloides, wherein said plasmid contains an origin of replication of pLeu4 and is capable of transforming said Rhizopus species and producing said target protein.

2. The host vector system of claim 1, wherein the Rhizopus species is selected from the group consisting of Rhizopus niveus, Rhizopus delemar, Rhizopus javanicus, Rhizopus oligosporus, Rhizopus nigricans and Rhizopus oryzae.

3. The host vector system of claim 2, wherein the Rhizopus species is Rhizopus niveus.

4. The host vector system of claim 3, wherein at least one plasmid is integrated into a chromosome of Rhizopus niveus.

5. The host vector system of claim 3, wherein at least one plasmid is extrachromosomal.

6. The host vector system of claim 3, wherein at least one plasmid is integrated into a chromosome of Rhizopus niveus and at least one plasmid is extrachromosomal.

7. The host vector system of claim 3, wherein the plasmid is pMCL1302, pMCL002, pMA67 or pLeu4.

## Patentansprüche

1. Wirt-Vektor-System für die Erzeugung eines Zielproteins in Rhizopus, wobei das System eine Rhizopus-Spezies und mindestens ein Mucor circinelloides-stämmiges Plasmid aufweist, wobei das Plasmid einen Replikationsstartpunkt von pLeu4 enthält und in der Lage ist, die Rhizopus-Spezies zu transformieren und das Zielprotein zu erzeugen.

2. Wirt-Vektor-System nach Anspruch 1, bei dem die Rhizopus-Spezies ausgewählt ist aus der Gruppe, die besteht aus Rhizopus niveus, Rhizopus delemar, Rhizopus javanicus, Rhizopus oligosporus, Rhizopus nigricans und Rhizopus oryzae.

3. Wirt-Vektor-System nach Anspruch 2, bei dem die Rhizopus-Spezies Rhizopus niveus ist.

4. Wirt-Vektor-System nach Anspruch 3, bei dem mindestens ein Plasmid in ein Chromosom von Rhizopus niveus eingefügt ist.

5. Wirt-Vektor-System nach Anspruch 3, bei dem mindestens ein Plasmid extrachromosomal ist.

6. Wirt-Vektor-System nach Anspruch 3, bei dem mindestens ein Plasmid in ein Chromosom von Rhizopus niveus eingefügt ist und mindestens ein Plamid extrachromosomal ist.

7. Wirt-Vektor-System nach Anspruch 3, bei dem das Plasmid pMCL1302, pMCL002, pMA67 oder pLeu4 ist.

## Revendications

1. Un système hôte-vecteur pour la production d'une protéine cible dans Rhizopus, ledit système comprenant une espèce Rhizopus et au moins un plasmide dérivé de circinelloïdes Mucor,
dans lequel ledit plasmide contient une origine de réplication de pLeu4 et est capable de transformer ladite espèce Rhizopus et de produire ladite protéine cible.

2. Le système hôte-vecteur de la revendication 1, dans lequel l'espèce Rhizopus est choisie dans le groupe constitué par Rhizopus niveus, Rhizopus delemar, Rhizopus javanicus, Rhizopus oligosporus, Rhizopus nigricans et Rhizopus oryzae.

3. Le système hôte-vecteur de la revendication 2, dans lequel l'espèce Rhizopus est Rhizopus niveus.

4. Le système hôte-vecteur de la revendication 3, dans lequel au moins un plasmide est introduit dans un chromosome de Rhizopus niveus.

5. Le système hôte-vecteur de la revendication 3, dans lequel au moins un plasmide est extra-chromosomique.

6. Le système hôte-vecteur de la revendication 3, dans lequel au moins un plasmide est introduit dans un chromosome de Rhizopus niveus et au moins un plasmide est extra-chromosomique.

7. Le système hôte-vecteur de la revendication 3, dans lequel le plasmide est pMCL1302, pMCL002, pMA67 ou pLeu4.
